# EUROPEAN PATENT SPECIFICATION

(11) **EP 1 429 746 B1**
(45) Date of publication and mention of the grant of the patent: **13.08.2008**
(21) Application number: 02799684.2
(22) Date of filing: 28.09.2002
(51) Int. Cl.: A61K 9/28

(54) **DOSAGE FORMS HAVING AN INNER CORE AND OUTER SHELL**
DARREICHUNGSFORMEN MIT KERN UND ÄUSSERER HÜLLE
FORME PHARMACEUTIQUE COMPRENANT UN NOYAU INTERNE ET UNE COQUE EXTERNE

(30) Priority: 28.09.2001 US 966939; 28.09.2001 US 966509; 28.09.2001 US 966497; 28.09.2001 US 967414; 28.09.2001 US 966450
(43) Date of publication of application: 23.06.2004
(73) Proprietor: McNEIL-PPC, Inc., Skillman, NJ 08558-9418 (US)
(72) Inventor: BUNICK, Frank, J., Quakertown, PA 18951 (US); SOWDEN, Harry, S., GLENSIDE, PA 19038 (US); THOMAS, Martin, Lake Worth, FL 33467 (US); BURKE, John, Jamison, PA 18929 (US); LEE, Der-Yang, Flemington, NJ 08822 (US)
(74) Representative: Fisher, Adrian John
(86) International application number: PCT/US2002/031066
(87) International publication number: WO 2003/026612

(56) References cited:
- EP-A- 0 279 682
- US-A- 5 314 696
- US-A- 5 415 868
- US-A- 5 824 338
- DATABASE ESPACENET EPO; results of search for "charge control agent" 4 September 2007 (2007-09-04),

## Description

### BACKGROUND OF THE INVENTION

### 1. Field of the Invention

This invention relates to a dosage form. More particularly, this invention relates to a dosage form having a core surrounded by a shell, such as a pharmaceutical composition having a core surrounded by a shell.

### 2. Background Information

A variety of dosage forms, such as tablets, capsules and gelcaps are known in the pharmaceutical arts. Tablets generally refer to relatively compressed powders in various shapes. Capsules are typically manufactured using a two piece gelatin shell formed by dipping a steel rod into gelatin so that the gelatin coats the end of the rod. The gelatin is hardened into two half-shells and the rod extracted. The hardened half-shells are then filled with a powder and the two halves joined together to form the capsule. (See generally, HOWARD C. ANGEL ET AL., Pharmaceutical Dosage Forms and Drug Delivery Systems (7th Ed. 1999).)

Film coated tablets are an improvement over uncoated tablets in terms of aesthetics, stability, and swallowability. One type of elongated, capsule-shaped film-coated tablet is commonly referred to as a "caplet." Typical film coatings have a thickness from about 5 to about 50 µm, and comprise various film forming polymers such as cellulose ethers and the like. Typically, such polymers are applied to the tablets either from solution in organic solvents, or from aqueous dispersion via conventional spraying methods such as those disclosed in U.S. Pat. Nos. 4,973,480 and U.S. 6,113,945. Conventional spray-coating processes do not confer a high level of surface gloss to the dosage forms, and film thickness is limited by both the film properties and processing costs. Additionally, it is not commercially feasible to spray-coat a tablet with a different color coating on each end.

Sugar coated tablets, such as those disclosed in U.S. Pat. Nos. 2,925,365; 3,420,931; 4,456,629; and 3,361,631, and particularly those which have been polished, for example, with a top coat of carnuba wax, may typically possess higher surface gloss and thicker coatings than film coated tablets, however the sugar coating process is highly time consuming and costly, and the coatings thus prepared can disadvantageously retard the dissolution of the dosage forms. While sugar coatings are typically thicker than film coatings, and can have the effect of rounding the tablet edges, the overall shape of a sugar-coated tablet depends upon and is substantially the same as that of the uncoated core.

Gelatin-coated tablets, commonly known as geltabs and gelcaps, are an improvement on gelatin capsules and typically comprise a tablet coated with a glossy gelatinous shell. Several well known examples of gelcaps are McNeil-PPC, Inc.'s acetaminophen based products sold under the trade name Tylenol®. One category of methods for producing such geltabs and gelcaps involve dipping tablets, one half at a time, into coating solutions, which can be of two different colors, see e.g. U.S. Patent Nos. 4,820,524, 5,538,125; 5,228,916; 5,436,026; 5,679,406; or dipping tablets of a first color halfway into a coating solution of a second color, see, e.g. U.S. Patent No. 6,113,945. U.S. Patent Nos. 5,942,034 and 6,195,911 describe additional methods and apparatuses for dip coating tablets. Another category of such methods involves shrink-fitting the capsule halves onto a tablet form. See, for example, U.S. Patent Nos. 5,415,868; 6126767; 6,080,426; 5,460,824; 5,464,631; 5,795,588; 5,511,361; 5,609,010; 6,245,350; and WO 97/37629. Another method of producing gelcaps is via an enrobing process wherein two separate films made of gelatinous material are applied to opposite sides of a tablet by a pair of rotary dies, as disclosed for example, in U.S. Patent Nos. 5,146,730 and 5,459,983.

Conventional methods for forming gelcaps are generally performed in a batchwise manner using a number of stand alone machines operating independently. Such batch processes typically include the unit operations of granulating, drying, blending, compacting (e.g., in a tablet press), film coating (e.g. by spraying in a coating pan), gelatin dipping, encapsulating or enrobing, drying, and printing.

Dipped gelcaps and geltabs may suffer from the limitations of variation in coating or shell thickness, and non-uniformity in color of the coating or shell.

Film formulations for producing gelcaps and geltabs prepared via enrobing methods such as those disclosed in U.S. Patent Nos. 5,146,730 and 5,459,983 typically comprises a water-based gelatin preparation having about 45% gelatin and about 9% plasticizer (glycerin and/or sorbitol) by weight. The plasticizer has been reported to play a critical role in such formulations. Low ratios of plasticizer to gelatin result in a brittle coating around the tablet core, while high ratios result in a gelatin coating around the tablet which is flexible and can be peeled from the tablet. If a gelatin coating which adheres to the product core is desired, then gelatin formulations having by-weight compositions of 40 percent to 60 percent gelatin, 5 percent to 12 percent plasticizer, 35 percent to 50 percent water, and colorants and pigments in the range of 0.1 percent to 3 percent should be considered. Glycerin and sorbitol can be used as single plasticizers or in combination with each other. In addition, other sugars and poly-hydroxy compounds can be used as additives and plasticizers. If a tamper-evident gelatin-coated medicine tablet is the desired end product, then the ratio of plasticizer to gelatin in the gelatin formulation should be in the range of about 1:5. The need for such plasticizers at such levels imparts limitations to enrobed dosage forms, including a propensity to absorb moisture, which may compromise the physical and chemical stability of the product, as well adding cost to the formulation.

Another current method for forming a shell (or coating), on a core (or substrate), is that disclosed in WO 01/57144 which utilizes the principles of electrostatic deposition to form the coating. This method suffers from the limitation that at least one of the core or the shell must incorporate one or more "charge control agents," such as metal salicylates, for example zinc salicylate, magnesium salicylate and calcium salicylate; quaternary ammonium salts; benzalkonium chloride; benzethonium chloride; trimethyl tetradecyl ammonium bromide (cetrimide); and cyclodextrins and their adducts, in an amount from about 1% to about 10% by weight of the shell. Charge control agents often cause an unpleasant taste sensation, and additionally may disadvantageously increase oxidation of the shell in which they are employed. It would therefore be advantageous to have a dosage form not employing charge control agents.

Other limitations shared by conventional encapsulation and enrobing processes include high cost and complexity, limitations on the thickness of the coating or shell, and the creation of raised seams between capsule halves and/or coatings. It would therefore be desirable to have dosage forms which were not prepared using conventional encapsulation or enrobing processes. Such dosage forms have enhanced versatility for a number of applications, including dosage forms to deliver pharmaceuticals, nutritionals and/or confections, which may be in the form of geltabs or gelcaps, coated tablets, high potency dosage forms and the like. Moreover, such dosage forms have unique and pleasant aesthetic qualities that are valuable in the marketplace.

It is one object of this invention to provide a dosage form comprising a core and a shell which surrounds and resides substantially conformally upon the core, such that the dosage form does not comprise any charge control agents.

It is another object of this invention to provide a dosage form comprising a core and a shell which surrounds the core, and the shell is free of any raised seam.

Other objects, features and advantages of this invention will be apparent to those skilled in the art from the detailed description of the invention provided herein.

### SUMMARY OF THE INVENTION

The dosage form of this invention is defined in claim 1. It comprises a core having an outer surface and a shell having outer and inner surfaces, wherein the shell surrounds the core, the dosage form moisture uptake after 60 minutes at 40°C and 75% relative humidity is less than 0.80%, the shell thickness is in the range of about 100-400 µm, the relative standard deviation of the shell thickness on the dosage form is less than 30%, the dosage form contains less than 1% charge control agents and the shell is free of a raised seam.

In another embodiment, the shell moisture uptake at 60 minutes of exposure to 40°C and 75% relative humidity is less than about 0.65%.

In another embodiment, the core comprises a compressed tablet.

In another embodiment, the dosage form has a belly band, and the difference in a first shell thickness at a first point on the belly band and a second thickness at a second point on the belly band is not more than about 10% of the larger of the two thicknesses.

In another embodiment, the dosage form has a belly band, and the difference in a first shell thickness at a first location on the belly band and a second thickness at a second location on the belly band is not more than about 50 microns.

In another embodiment, the shell surface is substantially free of a raised portion greater than about 1.25 times the shell thickness at a major face.

In another embodiment, the shell surface is substantially free of a raised portion greater than about 50 µm in height.

In another embodiment, the core has a major face, the dosage form has a belly band, and the shell thickness at any point on the belly band is not greater than the shell thickness at the center of a major tablet face.

In another embodiment, the core has a major face, the dosage form has a belly band, and the difference between the shell thickness at any point on the belly band and the shell thickness at the center of a major face is not greater than about 50 µm.

In another embodiment, the shell is substantially free of humectants.

In another embodiment, the core comprises an insert.

In another embodiment, the shell has a surface gloss value of at least about 150 gloss units.

In another embodiment, the shell has a surface gloss value of at least about 175 gloss units.

In another embodiment, the shell has a surface gloss value of at least about 210 gloss units.

In another embodiment, the core comprises an active ingredient.

In another embodiment, the active ingredient is capable of dissolution and the dissolution of the dosage form conforms to USP specifications for immediate release tablets containing the active ingredient.

In another embodiment, there is no subcoating located between the outer surface of the core and the inner surface of the shell.

In another embodiment, the shell comprises a first shell portion and a second shell portion which are joined at an interface.

In another embodiment, the first and second shell portions are visually distinct.

In another embodiment, the core is a tablet having a major face and the total thickness of the shell at the interface is not greater than the thickness of the thickest at the center of the major tablet face.

In another embodiment, the interface is an abutment.

In another embodiment, the first and second shell portions form an interlocking pattern at the interface.

In another embodiment, the first and second shell portions overlap one another.

In another embodiment, the interface of the first and second shell portions is a flat seam in which the transition from first shell portion to second shell portion lies along a single horizon line.

The inner surface of the shell has indentations and protrusions corresponding substantially inversely to indentations and protrusions on the outer surface of the core.

The indentations and protrusions have a length, width, height or depth greater than 10 µm.

In another embodiment, the shell is substantially free of pores having a pore diameter of 0.5-5.0 µm.

### BRIEF DESCRIPTION OF THE DRAWINGS

Figure 1 is a cross sectional view of an example of a dosage form of this invention.

Figure 2 depicts a dosage form and measurement positions for determining the relative standard deviation of the shell thickness.

Figures 3A and 3B are micrographs of commercially available prior art geltabs.

Figure 4 is a micrograph of the shell and core in one embodiment of this invention.

Figure 5 is a cross-sectional depiction of a core having major faces surrounded by a shell having a gap between the core and shell.

Figure 6 is an exploded view of the interface between the core and shell in Figure 5.

Figure 7A is a cross-sectional view of first and second shell portions which form an abutment without any overhang.

Figure 7B is a cross-sectional view of first and second shell portions which form an abutment with an overhang. (Not in accordance with the invention.)

Figures 8A-8E are cross-sectional views of first and second shell portions which overlap at the interface thereof.

Figures 9A-9F are cross-sectional views of first and second shell portions which overlap and form an interlocking pattern at the interface thereof.

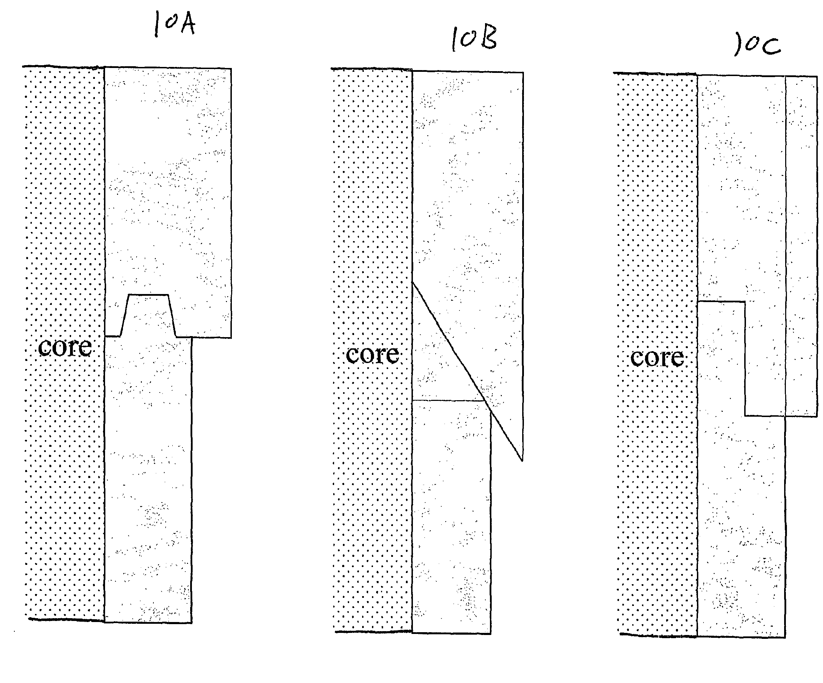

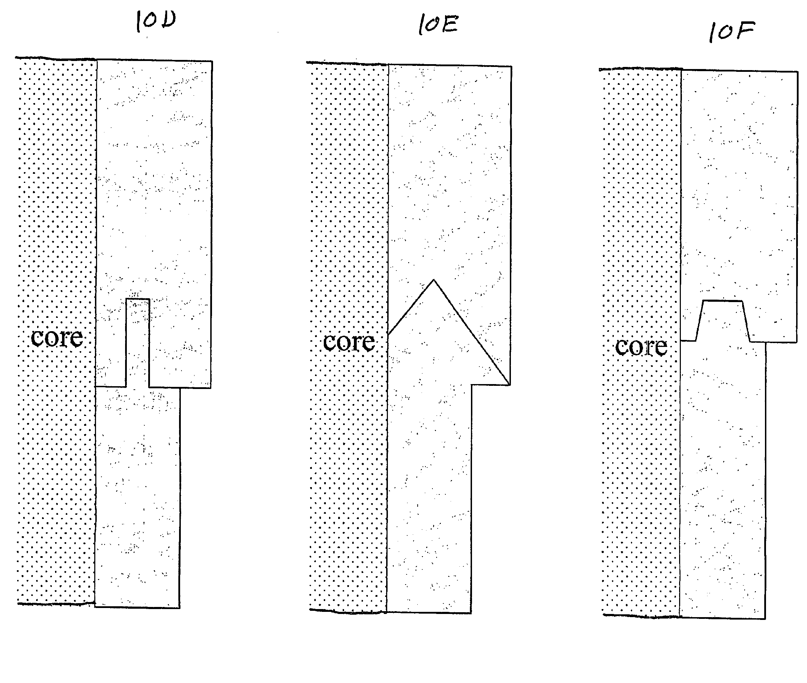

Figures 10A-10F are cross-sectional views of first and second shell portions which form an interface with an overhang. (Not in accordance with the invention.)

Figures 11A and 11B compare an injection molded dosage form of this invention and a prior art dosage form.

Figures 12A and 12B depict front and side views of a dosage form of this invention having a belly band.

Figure 13 is a plot of sample weight change vs. time depicting relative humidity data as described in Example 6 herein.

### DETAILED DESCRIPTION OF THE INVENTION

The dosage form of this invention comprises a core having an outer surface and a shell having outer and inner surfaces, and comprises further features as defined in claim 1. As used herein, the term "dosage form" applies to any solid object, semi-solid, or liquid composition, designed to contain a specific pre-determined amount (i.e. dose) of a certain ingredient, for example an active ingredient as defined below. Suitable dosage forms may be pharmaceutical drug delivery systems, including those for oral administration, buccal administration, rectal administration, topical, transdermal, or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human. In another preferred embodiment, the dosage form is an orally administered "placebo" system containing pharmaceutically inactive ingredients, and the dosage form is designed to have the same appearance as a particular pharmaceutically active dosage form, such as may be used for control purposes in clinical studies to test, for example, the safety and efficacy of a particular pharmaceutically active ingredient.

Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active agent may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active agent is selected from analgesics, anti-inflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In a particularly preferred embodiment, the active agent is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In a particular embodiment of the invention, the active agent may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active agent may be selected from pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260. As used herein, the term "simethicone" refers to the broader class of polydimethylsiloxanes, including but not limited to simethicone and dimethicone.

The active ingredient or ingredients may be present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dose regime, the age and weight of the patient, and other factors must be considered, as known in the art. In one embodiment, the core comprises at least about 85 weight percent of the active ingredient. In such an embodiment, the dosage form comprises at least about 85 weight percent of the active ingredient.

If embodiments in which modified release of the active ingredient is desired, the active ingredient may be coated with a release-modifying coating, as known in the art. Commercially available modified release active ingredients may also be employed. For example, acetaminophen particles which are encapsulated with release-modifying polymers by a coaccervation process may be used in the present invention. Coaccervation-encapsulated acetaminophen may be purchased commercially from Eurand America, Inc. (Vandalia, Ohio), or from Circa Inc. (Dayton, Ohio).

In embodiments in which the dosage form is intended to be chewed or disintegrated in the mouth prior to swallowing, the active ingredient may preferrably be coated with a taste-masking coating, as known in the art. Examples of suitable taste-masking coatings are described in U.S. Patent Nos. 4,851,226, 5,075,114, and 5,489,436. Commercially available taste-masked active ingredients may also be employed. For example, acetaminophen particles which are encapsulated with taste-masking polymers by a coaccervation process may be used in the present invention as described above.

Suitable tablet excipients include fillers, binders, disintegrants, lubricants, glidants, and the like.

Suitable fillers include water-soluble compressible carbohydrates such as sugars, which include dextrose, sucrose, maltose, and lactose, sugar-alcohols, which include mannitol, sorbitol, maltitol, xylitol, starch hydrolysates, which include dextrins, and maltodextrins, and the like, water insoluble plasticly deforming materials such as microcrystalline cellulose or other cellulosic derivatives, water-insoluble brittle fracture materials such as dicalcium phosphate, tricalcium phosphate and the like and mixtures thereof.

Suitable binders include dry binders such as polyvinyl pyrrolidone, hydroxypropylmethylcellulose, and the like; wet binders such as water-soluble polymers, including hydrocolloids such as alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan, polyvinyl pyrrolidone, cellulosics, starches, and the like; and derivatives and mixtures thereof.

Suitable disintegrants include sodium starch glycolate, cross-linked polyvinylpyrrolidone, cross-linked carboxymethylcellulose, starches, microcrystalline cellulose, and the like.

Suitable lubricants include long chain fatty acids and their salts, such as magnesium stearate and stearic acid, talc, and waxes.

Suitable glidants include colloidal silicon dioxide, and the like.

The dosage form of the invention may also incorporate pharmaceutically acceptable adjuvants, including, for example, preservatives, high-intensity sweeteners such as aspartame, acesulfame potassium, sucralose, and saccharin; flavors, antioxidants, surfactants, and coloring agents.

In embodiments in which it is desired for the active ingredient to be absorbed into the systemic circulation of an animal, the active ingredient or ingredients are preferably capable of dissolution upon contact with a fluid such as water, gastric fluid, intestinal fluid or the like. In one embodiment the dissolution characteristics of the active ingredient meet USP specifications for immediate release tablets containing the active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999). In another embodiment, the dissolution characteristics of the active ingredient are modified: e.g. controlled, sustained, extended, retarded, prolonged, delayed and the like.

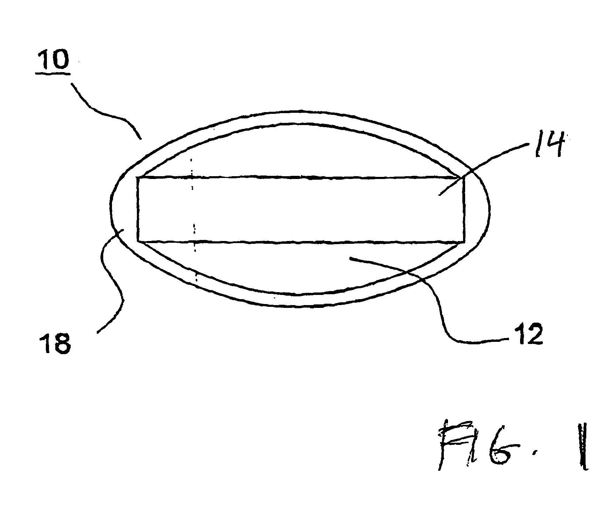

An overall understanding of the dosage form of this invention may be obtained by reference to Figure 1. In Figure 1, a dosage form 10 is depicted which comprises a molded shell 18 having a shape which surrounds the outside surface of a core 12 (which may be a molded core or a compressed dosage form or a hard or soft capsule, or any substantially solid edible form) having a different shape than the shell 18. Core 12 includes a belly band 14. It will be understood that the shapes of the core and shell in Figure 1 are illustrative,

The core or substrate may be any solid form. The core may be, for example, a compressed dosage form, or may be molded. As used herein, "substrate" refers to a surface or underlying support, upon which another substance resides or acts, and "core" refers to a material which is at least partially enveloped or surrounded by another material. The core may optionally comprise a sub-core (which may also be referred to as an "insert"), which may be made by any method, for example, compression or molding, and may optionally contain one or more active ingredients. For example, the core may be a compressed or molded tablet, hard or soft capsule, suppository, or a confectionery form such as a lozenge, nougat, caramel, fondant, or fat based form. In one embodiment, the core may comprise a microelectronic device (e.g. an electronic "chip") which may be used for any purpose, including for example as an active component or to detect environmental conditions or to control , for example, release of active ingredients from the dosage form or device.

The core may be in a variety of different shapes. For example, in one embodiment the core may be in the shape of a truncated cone. In other embodiments the core may be shaped as a polyhedron, such as a cube, pyramid, prism, or the like; or may have the geometry of a space figure with some non-flat faces, such as a cone, cylinder, sphere, torus, or the like. Exemplary core shapes which may be employed include tablet shapes formed from compression tooling shapes described by "The Elizabeth Companies Tablet Design Training Manual" (Elizabeth Carbide Die Co., Inc., p.7 (McKeesport, Pa.) as follows (the tablet shape corresponds inversely to the shape of the compression tooling):
1. Shallow Concave.
2. Standard Concave.
3. Deep Concave.
4. Extra Deep Concave.
5. Modified Ball Concave.
6. Standard Concave Bisect.
7. Standard Concave Double Bisect.
8. Standard Concave European Bisect.
9. Standard Concave Partial Bisect.
10. Double Radius.
11. Bevel & Concave.
12. Flat Plain.
13. Flat-Faced-Beveled Edge (F.F.B.E.).
14. F.F.B.E. Bisect.
15. F.F.B.E. Double Bisect.
16. Ring.
17. Dimple.
18. Ellipse.
19. Oval.
20. Capsule.
21. Rectangle.
22. Square.
23. Triangle.
24. Hexagon.
25. Pentagon.
26. Octagon.
27. Diamond.
28. Arrowhead.
29. Bullet.
30. Barrel.
31. Half Moon.
32. Shield.
33. Heart.
34. Almond.
35. House/Home Plate.
36. Parallelogram.
37. Trapezoid.

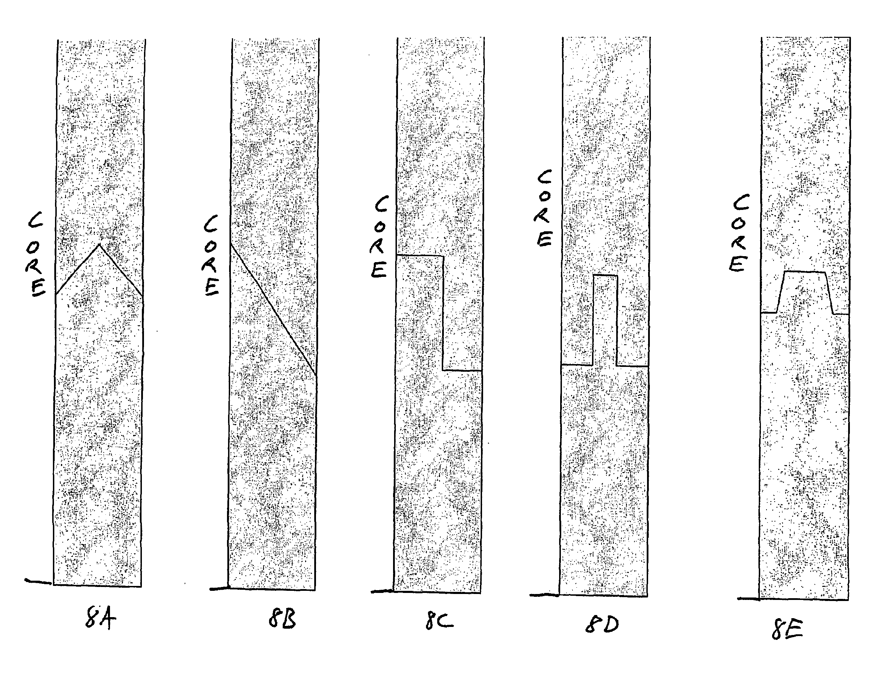

38. Figure 8/Bar Bell.
39. Bow Tie.
40. Uneven Triangle.

The core or sub-core may optionally be at least partially covered by a compressed, molded, or sprayed sub-coating. However, in one preferred embodiment, the core may be substantially free of the subcoating: i.e. there is no subcoating located between the outer surface of the core and the inner surface of the shell.

In another preferred embodiment of this invention, the core is a compressed dosage form, i.e. tablet, obtained from a compressed powder. The powder may preferably comprise an active ingredient, and optionally comprise various excipients, such as binders, disintegrants, lubricants, fillers and the like, as is conventional, or the powder may comprise other particulate material of a medicinal or non-medicinal nature, such as inactive placebo blends for tableting, confectionery blends, and the like. One particularly preferred formulation comprises active ingredient, powdered wax (such as shellac wax, microcrystalline wax, polyethylene glycol, and the like), and optionally disintegrants and lubricants and is described in more detail at pages 4-11 of copending United States Patent Application Serial No., 09/966,493.

In one embodiment of the invention, the dosage forms of this invention comprise a core made from a powder having an average particle size of about 50 to about 500 µm. In one embodiment, the active ingredient has an average particle size of about 50 to about 500 µm. In another embodiment, at least one excipient has an average particle size of about 50 to about 500 µm. In one such embodiment, a major excipient, i.e. and excipient comprising at least 50% by weight of the core, has an average particle size of about 50 to about 500 µm. Particles in this size range are particularly useful for direct compression processes.

In a preferred embodiment of the invention, the core may be prepared by a direct compression process. Using this technique, the compressed cores are produced by directly compacting a blend of the active agent and any other appropriate inactive ingredients, i.e., excipients.

Any conventional compacting methods for forming a solid dosage form may be used to make the core of the present invention. These methods include, but are not limited to, dry granulation followed by compression, and wet granulation followed by drying and compression. Compression methods include rotary compression, compacting roller technology, such as a chilsonator or drop roller, or by molding, casting, or extrusion technologies. These methods are well known in the art, and are described in detail in, for example, Lachman, et al., The Theory and Practice of Industrial Pharmacy, Chapter 11 (3rd Ed. 1986).

One such method utilizes placing a pre-determined volume of particles or components into a die cavity of a rotary tablet press, which continuously rotates as part of a die table from the filling position to a compaction position. At the compaction position, the particles are compacted between an upper punch and a lower punch. The die table then rotates to an ejection position, at which the resulting tablet is pushed from the die cavity by the lower punch and guided to an ejection chute by a stationary take-off bar.

In another embodiment of the invention, the core is a directly compressed tablet, made from a powder which is substantially free of water soluble polymeric binders and hydrated polymers. This composition is advantageous for maintaining an immediate release dissolution profile, minimizing processing and material costs, and providing for optimal physical and chemical stability of the dosage form.

In embodiments in which the core is prepared by direct compression, the materials comprising the core, e.g. active ingredient or ingredients and excipients, are blended together, preferably as dry powders, and fed into an apparatus that applies pressure and forms a core. Any suitable compacting apparatus may be used, including for example a roller compactor such as a chilsonator or drop roller; or a conventional tablet press. Preferably, the core is formed by compaction using a rotary tablet press as known in the art. In a rotary tablet press, a metered volume of powder is filled into a die cavity, which rotates as part of a "die table" from the filling position to a compaction position where the powder is compacted between an upper and a lower punch to an ejection position where the resulting tablet is pushed from the die cavity by the lower punch. The direct compression process enables the minimization or elimination of water-soluble, non-saccharide polymeric binders such as polyvinyl pyrrolidone, alginates, hydroxypropyl cellulose, hydroxypropylmethylcellulose, hydroxyethylcellulose, and the like, which can have an adverse effect on dissolution.

In another embodiment, the core is prepared by a wet-granulation method, in which the active ingredient, appropriate excipients, and a solution or dispersion of a wet binder (e.g an aqueous cooked starch paste, or solution of polyvinyl pyrrolidone) are mixed and granulated. Suitable apparatuses for wet granulation include low shear, e.g. planetary mixers, high shear mixers, and fluid beds, including rotary fluid beds. The resulting granulated material is dried, and optionally dry-blended with further ingredients, e.g. adjuvants and/or excipients such as for example lubricants, colorants, and the like. The final dry blend is then suitable for compression by the methods described above.

Methods for direct compression and wet granulation processes are known in the art, and are described in detail in, for example, Lachman, et al., The Theory and Practice of Industrial Pharmacy, Chapter 11 (3rd Ed. 1986).

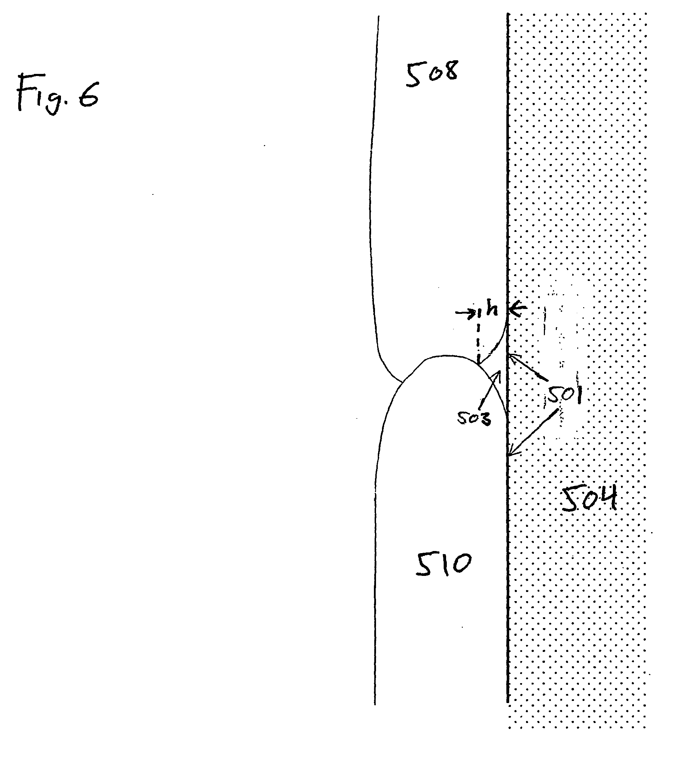

In another embodiment, the core is prepared by the compression methods and apparatus described in copending U.S. patent application Serial No. 09/966,509, pages 16-27.
Specifically, the core is made using a rotary compression module comprising a fill zone, insertion zone, compression zone, ejection zone, and purge zone in a single apparatus having a double row die construction as shown in Figure 6 of U.S. patent application Serial No. 09/966,509. The dies of the compression module are preferably filled using the assistance of a vacuum, with filters located in or near each die. The purge zone of the compression module includes an optional powder recovery system to recover excess powder from the filters and return the powder to the dies.

The core may alternatively be made by the thermal setting molding method and apparatus described in copending U.S. patent application Serial No. 09/966,450, pages 57-63.
In this embodiment, the core is formed by injecting a starting material in flowable form into a molding chamber. The starting material preferably comprises an active ingredient and a thermal setting material at a temperature above the melting point of the thermal setting material but below the decomposition temperature of the active ingredient. The starting material is cooled and solidifies in the molding chamber into a shaped core (i.e., having the shape of the mold).

According to this method, the starting material must be in flowable form. For example, it may comprise solid particles suspended in a molten matrix, for example a polymer matrix. The starting material may be completely molten or in the form of a paste. The starting material may comprise an active ingredient dissolved in a molten material. Alternatively, the starting material may be made by dissolving a solid in a solvent, which solvent is then evaporated from the starting material after it has been molded.

The starting material may comprise any edible material which is desirable to incorporate into a shaped form, including active ingredients, nutritionals, vitamins, minerals, flavors, sweeteners, and the like. Preferably, the starting material comprises an active ingredient and a thermal setting material. The thermal setting material may be any edible material that is flowable at a temperature between about 37 and about 120°C, and that is a solid at a temperature between about 0 and about 35°C. Preferred thermal setting materials include water-soluble polymers such as polyalkylene glycols, polyethylene oxides and derivatives, and sucrose esters; fats such as cocoa butter, hydrogenated vegetable oil such as palm kernel oil, cottonseed oil, sunflower oil, and soybean oil; mono-, di-, and triglycerides, phospholipids, waxes such as carnuba wax, spermaceti wax, beeswax, candelilla wax, shellac wax, microcrystalline wax, and paraffin wax; fat-containing mixtures such as chocolate; sugar in the form on an amorphous glass such as that used to make hard candy forms, sugar in a supersaturated solution such as that used to make fondant forms; low-moisture polymer solutions such as mixtures of gelatin and other hydrocolloids at water contents up to about 30% such as those used to make "gummi" confection forms. In a particularly preferred embodiment, the thermal setting material is a water-soluble polymer such as polyethylene glycol.

In another embodiment, the core may be a hollow or evacuated core. For example, the core may be an empty capsule shell. Alternatively, a hollow core may be prepared for example by molding. In one such method, flowable material is injected into a mold cavity, and the cavity is brought to a temperature at which the outer surface of the core (which is in contact with the mold) begins to solidify or set. The excess flowable material from the center of the core is then withdrawn from the mold using suitable means, for example a piston pump. Alternatively, an empty capsule is used as a sub-core, and a coating layer is formed thereon by methods known in the art such as for example spray-coating, dip-coating, or thermal cycle molding as described in copending U.S. patent application Serial No.09/966,497, pages 27-51.

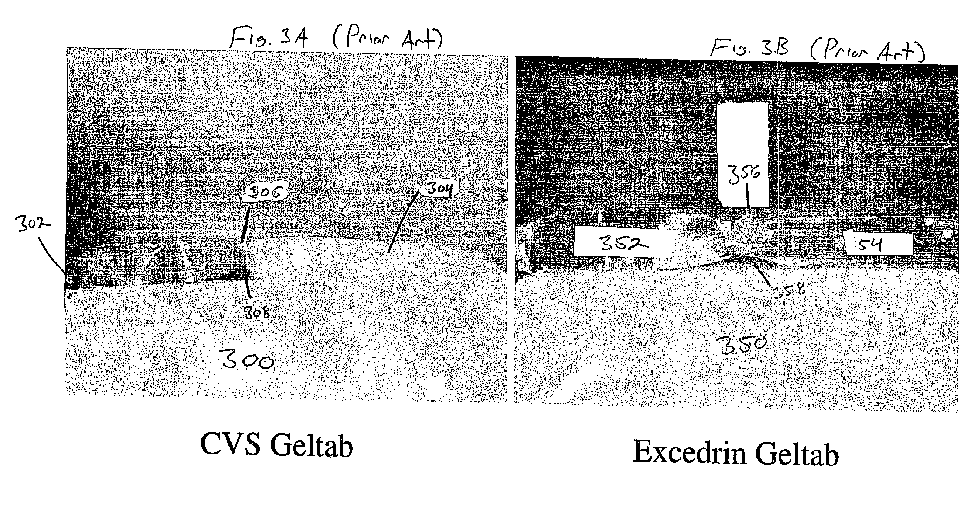

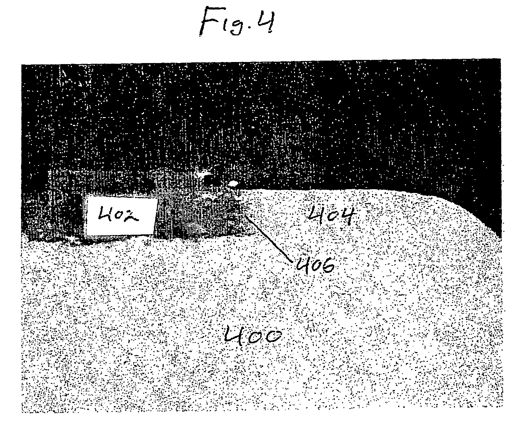

In the thermal cycle molding method and apparatus of U.S. patent application Serial No. 09/966,497, a thermal cycle molding module having the general configuration shown in Figure 3 therein is employed. The thermal cycle molding module 200 comprises a rotor 202 around which a plurality of mold units 204 are disposed. The thermal cycle molding module includes a reservoir 206 (see Figure 4) for holding flowable material to make the core. In addition, the thermal cycle molding module is provided with a temperature control system for rapidly heating and cooling the mold units. Figures 55 and 56 depict such a temperature control system 600.

In this embodiment, the mold units preferably comprise center mold assemblies 212 and upper mold assemblies 214 as shown in Figure 26C of U.S. patent application Serial No. 09/966,497, which mate to form mold cavities having the desired shape of the core. As rotor 202 rotates, the opposing center and upper mold assemblies close. Core flowable material, which is heated to a flowable state in reservoir 206, is injected into the resulting mold cavities. The temperature of the core flowable material is then decreased, hardening the core flowable material into cores. The mold assemblies open and eject the cores.

In another embodiment of the invention, the core is a compressed dosage form which contains one or more inserts. The inserts can be made in any shape or size. For instance, irregularly shaped inserts can be made, that is shapes having no more than one axis of symmetry. Cylindrically shaped inserts may also be made. In a preferred embodiment, the insert is prepared using the above described thermal setting method and apparatus described in copending U.S. patent application Serial No. 09/966,450, pages 57-63.

In one embodiment of the invention, the insert may have an average diameter from about 100 to about 1000 µm. In another embodiment of this invention, the insert may have an average diameter or thickness from about 10% to about 90% of the diameter or thickness of the core. In yet another embodiment of this invention, the core may comprise a plurality of inserts.

In another embodiment, the insert may have an average diameter, length, or thickness greater than about 90% of the diameter or thickness of the core, for example the insert may have an average length greater than about 100% of the thickness of the core.

The shell (or coating) of the present invention may comprise any material which can be molded, including for example, film formers, low-melting hydrophobic materials, gelling polymers, thickeners, plasticizers, adjuvants, and excipients. The shell comprises at least 80%, most preferably at least about 90% of a material selected from film formers, gelling polymers, low-melting hydrophobic materials, non-crystallizable sugars or sugar alcohols, and mixtures thereof. In another embodiment, the shell comprises at least about 50%, preferably at least about 80%, most preferably at least about 90% of a material selected from film formers, gelling polymers, low-melting hydrophobic materials, and mixtures thereof.

The shell is preferably made from a flowable material. The flowable material may be any edible material that is flowable at a temperature between about 37°C and 120°C, and that is solid or can form a gel at a temperature between about 0°C and about 35°C. When it is in the fluid or flowable state, the flowable material may comprise a dissolved or molten component, and a solvent such as for example water. The solvent may be partially or substantially removed by drying. Suitable flowable materials include those comprising film formers, gelling polymers, hydrocolloids, low melting hydrophobic materials such as fats and waxes, non-crystallizable sugars, and the like.

In one embodiment of the invention, the flowable material comprises gelatin. Gelatin is a natural, thermogelling polymer. It is a tasteless and colorless mixture of derived proteins of the albuminous class which is ordinarily soluble in warm water. Two types of gelatin - Type A and Type B - are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a 13mm (half-inch) diameter plastic plunger 4 mm into a 6.67% gelatin gel that has been held at 10°C for 17 hours. In a preferred embodiment, the flowable material is an aqueous solution comprising 20% 275 Bloom pork skin gelatin, 20% 250 Bloom Bone Gelatin, and approximately 60% water.

Other preferred flowable materials may comprise sucrose-fatty acid esters; fats such as cocoa butter, hydrogenated vegetable oil such as palm kernel oil, cottonseed oil, sunflower oil, and soybean oil; mono- di- and triglycerides, phospholipids, waxes such as carnuba wax, spermaceti wax, beeswax, candelilla wax, shellac wax, microcrystalline wax, and paraffin wax; fat-containing mixtures such as chocolate; sugar in the form on an amorphous glass such as that used to make hard candy forms, sugar in a supersaturated solution such as that used to make fondant forms; carbohydrates such as sugar-alcohols (for example, sorbitol, maltitol, mannitol, xylitol), or thermoplastic starch; and low-moisture polymer solutions such as mixtures of gelatin and other hydrocolloids at water contents up to about 30%, such as for example those used to make "gummi" confection forms.

In one preferred embodiment of the invention, the flowable material may comprise a film former such as a cellulose ether, e.g. hydroxypropylmethylcellulose or a modified starch, e.g. waxy maize starch; optionally an extender, such as polycarbohydrates, e.g. maltodextrin; optionally a thickener, such as a hydrocolloid, e.g. xanthan gum or carrageenan, or a sugar, e.g. sucrose; optionally a plasticizer, e.g. polyethylene glycol, propylene glycol, vegetable oils such as castor oil, glycerin, and mixtures thereof.

Any film former known in the art is suitable for use in the flowable shell material of the present invention. Examples of suitable film formers include, but are not limited to, polyvinylalcohol (PVA), polyvinylpyrrolidone (PVP), hydroxypropyl starch, hydroxyethyl starch, pullulan, methylethyl starch, carboxymethyl starch, methylcellulose, hydroxypropylcellulose (HPC), hydroxyethylmethylcellulose (HEMC), hydroxypropylmethylcellulose (HPMC), hydroxybutylmethylcellulose (HBMC), hydroxyethylethylcellulose (HEEC), hydroxyethylhydroxypropylmethyl cellulose (HEMPMC), methacrylic acid and methacrylate ester copolymers, polyethylene oxide and polyvinylpyrrolidone copolymers, gelatin, proteins such as whey protein, coaggulatable proteins such as albumin, casein, and casein isolates, soy protein and soy protein isolates, pre-gelatinized starches, and polymers and derivatives and mixtures thereof.

One suitable hydroxypropylmethylcellulose compound is "HPMC 2910", which is a cellulose ether having a degree of substitution of about 1.9 and a hydroxypropyl molar substitution of 0.23, and containing, based upon the total weight of the compound, from about 29% to about 30% methoxyl and from about 7% to about 12% hydroxylpropyl groups. HPMC 2910 is commercially available from the Dow Chemical Company under the tradename, METHOCEL E. METHOCEL E5, which is one grade of HPMC-2910 suitable for use in the present invention, has a viscosity of about 4 to 6 cps (4 to 6 millipascal-seconds) at 20°C in a 2% aqueous solution as determined by a Ubbelohde viscometer. Similarly, METHOCEL E6, which is another grade of HPMC-2910 suitable for use in the present invention, has a viscosity of about 5 to 7 cps (5 to 7 millipascal-seconds) at 20°C in a 2% aqueous solution as determined by a Ubbelohde viscometer. METHOCEL E15, which is another grade of HPMC-2910 suitable for use in the present invention, has a viscosity of about 15000 cps (15 millipascal-seconds) at 20°C in a 2% aqueous solution as determined by a Ubbelohde viscometer. As used herein, "degree of substitution" shall mean the average number of substituent groups attached to a anhydroglucose ring, and "hydroxypropyl molar substitution" shall mean the number of moles of hydroxypropyl per mole anhydroglucose.

As used herein, "modified starches" include starches that have been modified by crosslinking, chemically modified for improved stability, or physically modified for improved solubility properties. As used herein, "pre-gelatinized starches" or "instantized starches" refers to modified starches that have been prewetted, then dried to enhance their cold-water solubility. Suitable modified starches are commercially available from several suppliers such as, for example, A.E. Staley Manufacturing Company, and National Starch & Chemical Company. One suitable modified starch includes the pre-gelatinized waxy maize derivative starches that are commercially available from National Starch & Chemical Company under the tradenames, PURITY GUM and FILMSET, and derivatives, copolymers, and mixtures thereof. Such waxy maize starches typically contain, based upon the total weight of the starch, from about 0 percent to about 18 percent of amylose and from about 100% to about 88% of amylopectin.

Suitable tapioca dextrins include those available from National Starch & Chemical Company under the tradename, CRYSTAL GUM or K-4484, and derivatives thereof such as modified food starch derived from tapioca, which is available from National Starch and Chemical under the tradename, PURITY GUM 40, and copolymers and mixtures thereof.

Any thickener known in the art is suitable for use in the film forming composition of the present invention. Examples of such thickeners include but are not limited to hydrocolloids (also referred to herein as gelling polymers) such as alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan, and derivatives and mixtures thereof. Additional suitable thickeners include crystallizable sugars, such as glucose (dextrose), fructose, and the like, and derivatives and combinations thereof.

Suitable xanthan gums include those available from C.P. Kelco Company under the tradename, KELTROL 1000, XANTROL 180, or K9B310.

Any plasticizer known in the pharmaceutical art is suitable for use in the present invention, and may include, but not be limited to polyethylene glycol; glycerin; sorbitol; triethyl citrate; tribuyl citrate; dibutyl sebecate; vegetable oils such as castor oil; surfactants such as polysorbates, sodium lauryl sulfates, and dioctylsodium sulfosuccinates; propylene glycol; mono acetate of glycerol; diacetate of glycerol; triacetate of glycerol; natural gums and mixtures thereof. In solutions containing a cellulose ether film former, an optional plasticizer may be present in an amount, based upon the total weight of the solution, from about 0% to about 40%.

The flowable material may optionally comprise adjuvants or excipients, in which may comprise up to about 20% by weight of the flowable material. Examples of suitable adjuvants or excipients include detackifiers, humectants, surfactants, anti-foaming agents, colorants, flavorants, sweeteners, opacifiers, and the like. In one preferred embodiment, the flowable material comprises less than 5% humectants, or alternately is substantially free of humectants, such as glycerin, sorbitol, maltitol, xylitol, or propylene glycol. Humectants have traditionally been included in pre-formed films employed in enrobing processes, such as that disclosed in U.S. Patent Nos. 5,146,730 and 5,459,983, assigned to Banner Gelatin Products Corp., in order to ensure adequate flexibility or plasticity and bondability of the film during processing. Humectants function by binding water and retaining it in the film. Pre-formed films used in enrobing processes can typically comprise up to 45% water. Disadvantageously, the presence of humectant prolongs the drying process, and can adversely affect the stability of the finished dosage form.

The finished shell of the dosage form contains at least 80%; e.g. at least about 90% of a material selected from film formers, gelling polymers (hydrocolloids), low-melting hydrophobic materials, non-crystallizable sugars, and mixtures thereof. The shell of the present invention is formed by injection molding, advantageously minimizing or eliminating the need for direct-compression filler binders such as microcrystalline cellulose, spraydried lactose, mineral salts such as calcium phosphate, crystalline sugars such as sucrose, dextrates and the like. These materials would disadvantageously detract from the clarity and stability of the shell. Preferably the shell of the present invention comprises less than about 10%, e.g. less than about 1%, or less than about 0.1% of direct-compression filler-binders. The shells of the present invention are thus an improvement over compression-coated shells, which typically comprise at least about 30% of a direct-compression filler-binder. See for example, WO 00/18447.

In one embodiment, the shell is substantially free of humectants, such as for example glycerin and/or sorbitol, i.e. the shell contains less than about 5%, e.g. less than about 1%, or less than about 0.1% of a humectant. In another embodiment, the shell contains less than about 5%, e.g. less than about 1% of hygroscopic materials selected from crystallizable sugars, sugar alcohols, and glycerin. In another embodiment, the shell contains less than about 5% of a plasticizer.

The molded shell or a portion thereof is preferably substantially free of pores having a diameter of 0.5-5.0 µm. As used herein, "substantially free" means that the shell has a pore volume of less than about 0.02 cm³/g, preferably less than about 0.01 cm³/g, more preferably less than about 0.005 cm³/g, in the pore diameter range of 0.5 to 5.0 µm. Typical compressed materials have pore volumes of more than about 0.02 cm³/g in this pore diameter range. The pore volume may be determined using a Quantachrome Instruments PoreMaster 60 mercury intrusion porosimeter and associated computer software program known as "Porowin." The procedure is documented in the Quantachrome Instruments PoreMaster Operation Manual. The PoreMaster determines both pore volume and pore diameter of a solid or powder by forced intrusion of a non-wetting liquid (mercury), which involves evacuation of the sample in a sample cell (penetrometer), filling the cell with mercury to surround the sample with mercury, applying pressure to the sample cell by: (i) compressed air (up to 345kPa (50 psi) maximum); and (ii) a hydraulic (oil) pressure generator (up to 413685kPa (60000 psi) maximum). Intruded volume is measured by a change in the capacitance as mercury moves from outside the sample into its pores under applied pressure. The corresponding pore size diameter (d) at which the intrusion takes place is calculated directly from the so-called "Washburn Equation": d= -(4γ(cosθ))/P where γ is the surface tension of liquid mercury, θ is the contact angle between mercury and the sample surface and P is the applied pressure.

Equipment used for pore volume measurements:
(1) Quantachrome Instruments PoreMaster 60.
(2) Analytical Balance capable of weighing to 0.0001g.
(3) Desiccator.

Reagents used for measurements:
(1) High purity nitrogen.
(2) Triply distilled mercury.
(3) High pressure fluid (Dila AX, available from Shell Chemical Co.).
(4) Liquid nitrogen (for Hg vapor cold trap).
(5) Isopropanol or methanol for cleaning sample cells.
(6) Liquid detergent for cell cleaning.

Procedure:
The samples remain in sealed packages or as received in the dessicator until analysis. The vacuum pump is switched on, the mercury vapor cold trap is filled with liquid nitrogen, the compressed gas supply is regulated at 379kPa (55 psi), and the instrument is turned on and allowed a warm up time of at least 30 minutes. The empty penetrometer cell is assembled as described in the instrument manual and its weight is recorded. The cell is installed in the low pressure station and "evacuation and fill only" is selected from the analysis menu, and the following settings are employed:
   Fine Evacuation time: 1 min.
   Fine Evacuation rate: 10
   Coarse Evacuation time: 5 min.

The cell (filled with mercury) is then removed and weighed. The cell is then emptied into the mercury reservoir, and two tablets from each sample are placed in the cell and the cell is reassembled. The weight of the cell and sample are then recorded. The cell is then installed in the low-pressure station, the low-pressure option is selected from the menu, and the following parameters are set:
Mode: Low pressure
Fine evacuation rate: 10
Fine evacuation until: 200µ Hg
Coarse evacuation time: 10 min.
Fill pressure: Contact +0.1
Maximum pressure: 50
Direction: Intrusion And Extrusion
Repeat: 0
Mercury contact angle; 140
Mercury surface tension: 480

Data acquisition is then begun. The pressure vs. cumulative volume-intruded plot is displayed on the screen. After low-pressure analysis is complete, the cell is removed from the low-pressure station and reweighed. The space above the mercury is filled with hydraulic oil, and the cell is assembled and installed in the high-pressure cavity. The following settings are used:
Mode: Fixed rate
Motor speed: 5
Start pressure: 20
End pressure: 60 000
Direction: Intrusion and extrusion
Repeat: 0
Oil fill length: 5
Mercury contact angle: 140
Mercury surface tension: 480

Data acquisition is then begun and graphic plot pressure vs. intruded volume is displayed on the screen. After the high pressure run is complete, the low- and high-pressure data files of the same sample are merged.

In a preferred embodiment of the invention, the shell is applied to the core in the form of a flowable material using the thermal cycle method and apparatus described in copending U.S. patent application Serial No. 09/966,497, pages 27-51, In this embodiment, the shell is applied using a thermal cycle molding module having the general configuration shown in Figure 3 therein. The thermal cycle molding module 200 comprises a rotor 202 around which a plurality of mold units 204 are disposed. The thermal cycle molding module includes a reservoir 206 (see Figure 4 therein) for holding shell flowable material. In addition, the thermal cycle molding module is provided with a temperature control system for rapidly heating and cooling the mold units. Figures 55 and 56 depict the temperature control system 600.

The thermal cycle molding module is preferably of the type shown in Figure 28A of copending U.S. Application Serial No. 09/966,497, comprising a series of mold units 204. The mold units 204 in turn comprise upper mold assemblies 214, rotatable center mold assemblies 212 and lower mold assemblies 210 as shown in Figure 28C. Cores are continuously transferred to the mold assemblies, which then close over the cores. The shell flowable material, which is heated to a flowable state in reservoir 206, is injected into the mold cavities created by the closed mold assemblies. The temperature of the shell flowable material is then decreased, hardening it The mold assemblies open and eject the coated cores. Coating is performed in two steps, each half of the cores being coated separately as shown in the flow diagram of Figure 28B of copending U.S. Application Serial No. 09/966,497 via rotation of the center mold assembly.

In one embodiment, the shell of the present invention advantageously preferably has a high surface gloss which is a measure of reflected light determined according to the method set forth in Example 5 herein. The surface gloss of the shell and/or finished dosage form is preferably at least about 150 gloss units, e.g. at least about 175 gloss units, or at least about 210 gloss units. Dosage forms with high surface gloss are preferred by consumers due to their aesthetic elegance and perceived swallowability. The surface gloss of the shell depends upon a number of factors, including the shell composition, the method of forming the shell, and, if a mold is used, the surface finish on the mold.

One or more active ingredients may be contained in the dosage form of the invention in the core, the shell, the insert, or any combination thereof. In one embodiment of the invention, only the core comprises one or more active ingredients. In another embodiment of this invention, only the shell comprises one or more active ingredients. In yet another embodiment of this invention, only the insert comprises one or more active ingredients. In yet another embodiment of this invention, both the core and shell comprise one or more active ingredients. In yet another embodiment of this invention, one or more of the core, the shell, or the insert comprises one or more of the active ingredients.

AS used herein, the term "charge control agents" refers to a material having a charge control function, such as those used for electrostatic deposition of coatings onto substrates. Such charge control agents include metal salicylates, for example zinc salicylate, magnesium salicylate and calcium salicylate; quaternary ammonium salts; benzalkonium chloride; benzethonium chloride; trimethyl tetradecyl ammonium bromide (cetrimide); and cyclodextrins and their adducts.

In this embodiment of the invention, the shell comprises less than about 50%, preferably less than about 25%, most preferably less than about 5% of a crystallizable sugar.

As used herein, the term "substantially conformally" shall mean that the inner surface of the shell has peaks and valleys or indentations and protrusions corresponding substantially inversely to the peaks and valleys of the outer surface of the core. Accordingly, the indentations and protrusions typically have a length, width, height or depth in one dimension of greater than 10 µm, say greater than 20 µm, and less than about 30,000 µm, preferably less than about 2000 µm.

In one embodiment of this invention, the ratio of the height of the greatest gap between the core outer surface and the shell inner surface to the overall theoretical shell thickness at the point of the greatest gap is less than about 1:7.5, more preferably less than about 1:10, most preferably less than about 1:100.

In another embodiment, the ratio of the height of the greatest gap between the core outer surface and the shell inner surface to the shell thickness at the center of a major face is less than about 1:7.5, more preferably less than about 1:10, most preferably less than about 1:100. The term "major face," as used herein, shall apply to all cores, including but not limited to those which are compressed tablets. In one embodiment of this invention, the core has one or more major faces. For example, the core may be a polyhedron, such as a cube, pyramid prism or the like; or the core may have the geometry of a space figure with some non-flat faces, such as a cone, cylinder, sphere, torus or the like.

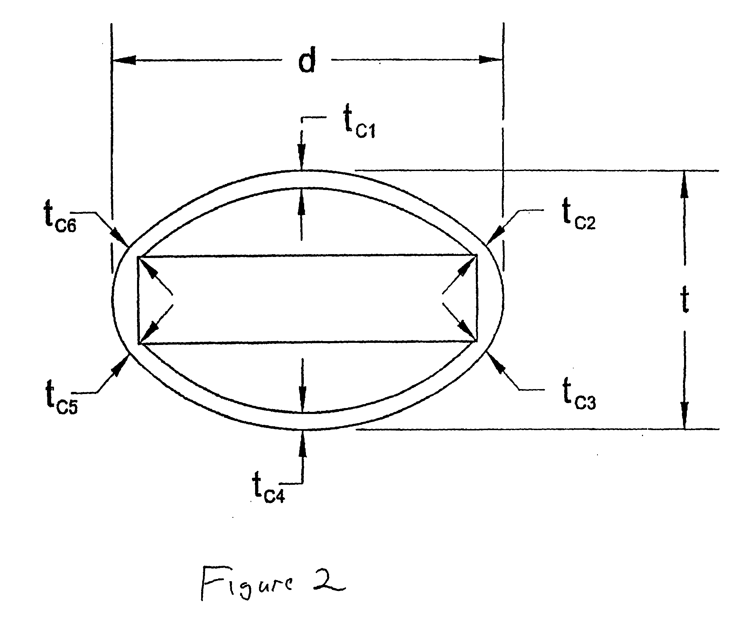

The shell thickness may be measured using a microscope, for example, an environmental scanning electron microscope, model XL 30 ESEM LaB6, Philips Electronic Instruments Company, Mahwah, WI. The shell thickness is measured at 6 different locations on a single dosage form, as shown in Figure 2. The relative standard deviation (RSD) is calculated as the sample standard deviation, devided by the mean, times 100 as known in the art (i.e. the RSD is the standard deviation expressed as a percentage of the mean). The RSD in shell thickness provides an indication of the variation in the thickness of the shell on a single dosage form. A uniform shell thickness is advantageous for providing aesthetic benefits such as for example uniformity of color, and uniform coverage of the substrate; and is particularly advantageous for embodiments in which the shell provides functional benefits, auch as for example modified release of an active ingredient contained within the dosage form. Advantageously the relative standard deviation in shell thickness for dosage forms of the present invention is preferably less than about 40%, e.g less than about 30%, or less than about 20%.
Location 1: center of first major face, t_{c1}
Locations 2 and 3: edges (near punch land) of intersection between first major face and side, t_{c2} and t_{c3}
Location 4: center of second major face, t_{c4}
Locations 5 and 6: edges (near punch land) of intersection between second major face and side, t_{c5} and t_{c6}

To determine RSD in overall dosage form thickness among a plurality of dosage forms, overall dosage form thickness and diameter are measured for 20 dosage forms using a calibrated electronic digital caliper. For thickness, the caliper is positioned across t as shown in Figure 2. For diameter, the caliper is positioned at the midsections of the widest point of the dosage form sides shown in Figure 2 as d.

The relative standard deviation of the thickness of the overall dosage form is less than 30%.
A high consistency of product characteristics, particularly overall dimensions, are considered advantageous in the pharmaceutical arts as an indication of high product quality and high degree of reliability, repeatability, reproducability and control in the manufacturing process. Consistency in product dimensions is additionally advantageous for the efficiency of downstream operations such as packaging, for example into blister packages, or into bottles via slat-filling machines.

Figure 3A is a micrograph cross-section of a prior art geltab sold by CVS pharmacies As shown in Figure 3A, the geltab comprises a core 300 having a shell made up of two portions 302 and 304 which interface and abut at 306. Proximate to interface 306 is a gap 308 between shell portions 302, 304 and the outer surface of core 300. Similarly, Figure 3B is a micrograph cross-section of a prior art EXCEDRIN geltab (a product of Bristol-Myers Squibb Co.). As shown in Figure 3B, the geltab comprises a core 350 having a shell made up of two portions 352 and 354 which interface and abut at 356. Proximate to interface 356 is a gap 358 between shell portions 352, 354 and the outer surface of core 350.

Figure 4 is a micrograph cross-section of a geltab dosage form of the present invention. As shown in Figure 4, the geltab comprises a core 400 having a shell made up of two portions 402 and 404 which interface and abut at 406. However, unlike the prior art geltabs shown in Figures 3A and 3B, the geltab of this invention as shown in Figure 4 has the shell portions 402 and 404 residing substantially conformally upon the outer surface of core 400 without a gap proximate to interface 406.

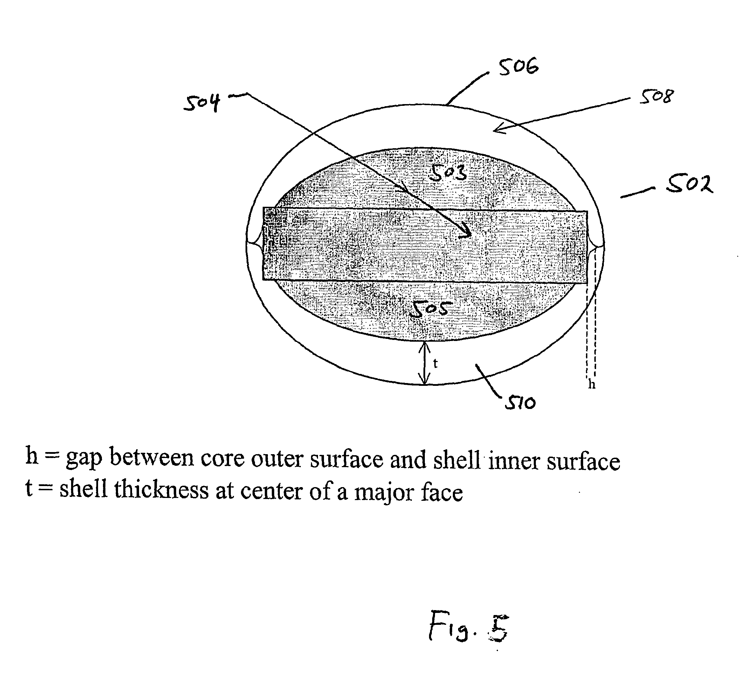

Figure 5 is representative of a cross-sectional depiction of a dosage form 502 comprising a core 504 having major faces 503 and 505 and a shell 506 made up of separate shell portions 508 and 510, respectively. As shown in Figure 5, "h" is the maximum gap between the core outer surface and the shell inner surface, and "t" is the shell thickness at the center of a major face. Accordingly, in the embodiment of the invention discussed above, h/t < 1:7.5, more preferably less than about 1:10, most preferably less than about 1:100.

Figure 6 depicts an exploded view of the interface between the core and shell of Figure 5. More specifically, Figure 6 depicts core 504 having an outer surface 501 which interfaces shell portions 508 and 510 as shown. The maximum gap 503 between outer surface 501 and the inner surface of the shell is shown by "h."

In contrast to dosage forms prepared by conventional enrobing processes such as those described in U.S. Patent Nos. 5,146,730 and 5,459,983, the shells of this embodiment present invention may advantageously be formed from relatively non-hydroscopic materials, which possess superior stability to elevated humidity conditions. Preferably, the dosage forms of the present invention have a moisture uptake at 10 minutes of exposure to 40°C/75% RH of less than about 0.35%, more preferably less than about 0.30%. Alternatively, the dosage forms of the present invention preferably have a moisture uptake at 20 minutes of exposure to 40°C/75% RH of less than about 0.50%, more preferably less than about 0.40%. Alternatively, the dosage forms of the present invention preferably have a moisture uptake at 30 minutes of exposure to 40°C/75% RH of less than about 0.60%, more preferably less than about 0.45%. Alternatively, the dosage forms of the present invention preferably have a moisture uptake at 60 minutes of exposure to 40°C/75% RH of less than about 0.80%, more preferably less than about 0.65%, when measured by the method set forth in Example 6 herein.

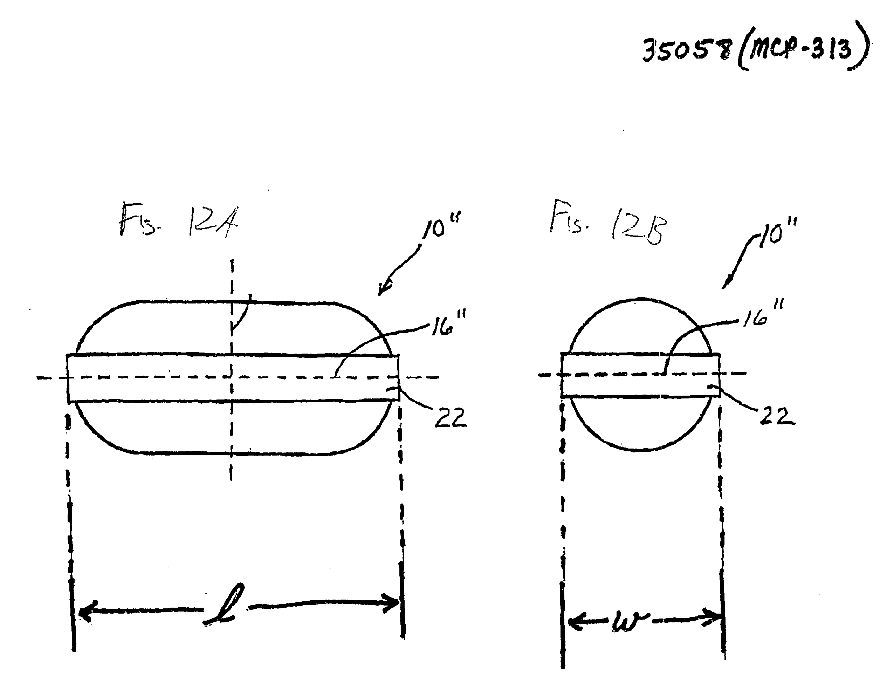

In embodiments wherein the core comprises a compressed tablet, the core and/or the finished dosage form typically comprises a "belly band," and two opposing faces which may be flat or curved as shown in Figures 12A and 12B. Figure 12A shows a front view of a dosage form 10" having an axial horizontal centerline 16" which is the midpoint of belly band 22. The length ("1") of the dosage form is shown in Figure 12A, and the width ("w") of the dosage form is shown in Figure 12B.

In one preferred embodiment if the dosage form has a belly band, the difference in a first shell thickness at a first point on the belly band and a second thickness at a second point on the belly band is not more than about 10% of the larger of the two thicknesses.

In another preferred embodiment, if the dosage form has a belly band, the shell thickness at any point on the belly band is not greater than the shell thickness at the center of a major tablet face.

In another preferred embodiment, the shell is substantially free of a raised portion greater than about 1.25 times the shell thickness at a major face.

In another preferred embodiment, the dosage form has a belly band, and the difference in a first shell thickness at a first location on the belly band and a second thickness at a second location on the belly band is not more than about 50 µm.

In another preferred embodiment, the shell surface is substantially free of a raised portion greater than about 50 µm in height.

In another preferred embodiment, the core has a major face, the dosage form has a belly band, and the difference between the shell thickness at any point on the belly band and the shell thickness at the center of a major face is not greater than about 50 µm.

In another embodiment of this invention, the shell comprises a first shell portion and a second shell portion. In one such embodiment, the first and second shell portions may comprise different shell materials. In another such embodiment, the first and second shell materials may be visually distinct from one another, for example the visually distinct portions may be of different colors, hues, glosses, reflective qualities, brightness, depth, shades, chroma, opacity, etc. For example, the shell may have a red portion and a yellow portion, or a flat finish portion and a glossy portion, or an opaque portion and a translucent portion.

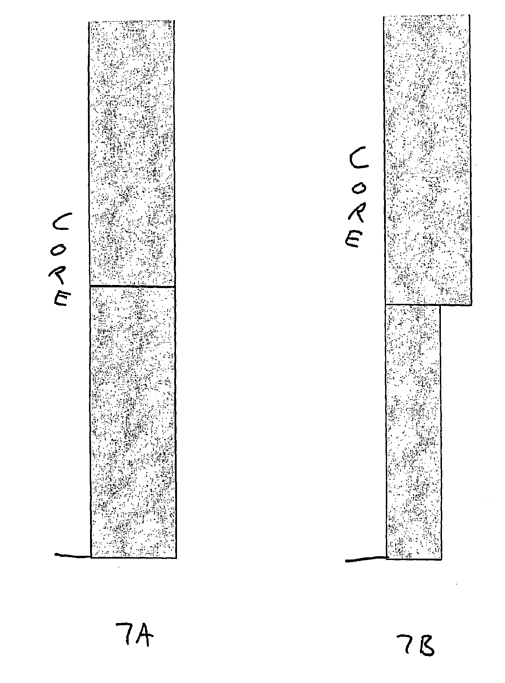

One advantage of the present invention is the ability to deposit more than one shell material onto a single core. In one embodiment wherein the shell comprises a first and second portion, the first and second shell portions are joined at an interface. In one such embodiment, the interface may be substantially flat (i.e., without any raised portion). In another such embodiment, the interface may be in the form of an abutment, i.e., the edges of the first and second portions are adjacent and in contact with one another, but not overlapping, as shown in Figure 7A.

In one particular embodiment in which the shell comprises a first and second portion which are joined at an interface, the interface is substantially flat (i.e., without any raised portion). In one such embodiment, in which the first and second shell thicknesses are substantially the same, the total shell thickness at the interface is not substantially different from the shell thickness of the first or second shell portions at the center of a major face of the dosage form. In another particular embodiment the shell comprises a first and second portion which are joined at an interface, the interface is substantially flat (i.e., without any raised portion), and the interface is located along the belly-band of the dosage form. In such embodiments, the total shell thickness at the interface is not substantially different from the shell thickness of the first or second shell portions at any point along the belly band of the dosage form.

In another embodiment, the first and second shell portions may overlap one another at the interface. The overlap may form various patterns, such as, for example, pointed, diagonal, step, tongue and groove, or truncated, as depicted in Figs. 8A-8E.

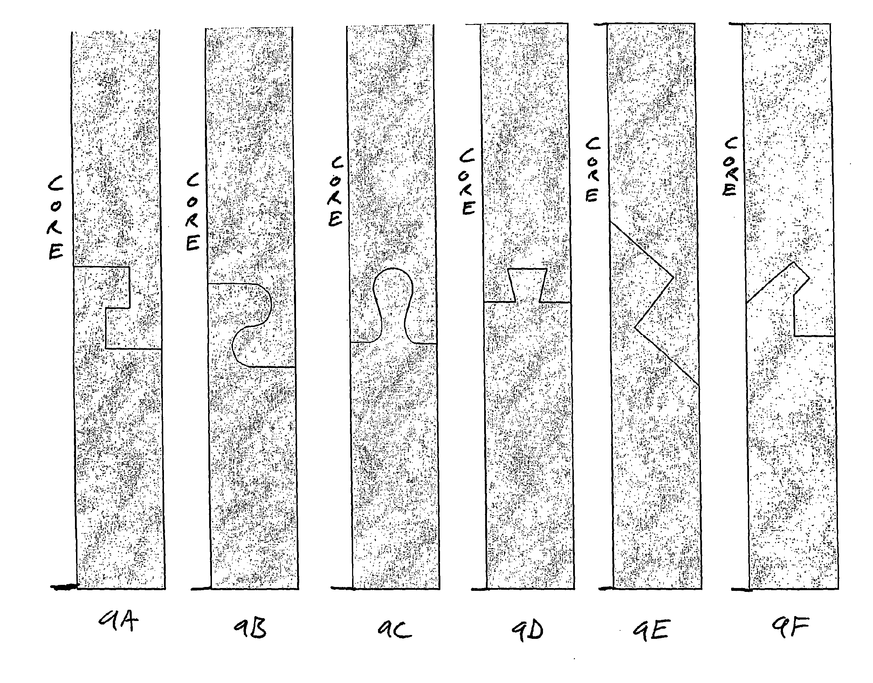

In yet another such embodiment, the first and second shell portions may overlap and form an interlocking pattern at the interface. The interlocking pattern may take various forms, such as, for example, a square interlock, various jigsaw "puzzle piece" configurations, a dove tail, a zigzag, or any number of complex asymmetric forms as depicted in Figures 9A-9F.

In one particular embodiment in which the shell comprises a first and second portion overlapping one another at an interface, the total shell thickness at the overlap is not greater than the median thickness of each single shell portion, so that the interface does not constitute a raised portion. In such embodiments, the total shell thickness at the interface is not greater than the shell thickness of the first or second shell portions at the center of a major face of the dosage form. In another particular embodiment in which the shell comprises a first and second portion overlapping one another at an interface, the total shell thickness at the interface is not greater than the shell thickness of the first or second shell portions at any point along the belly band of the dosage form.

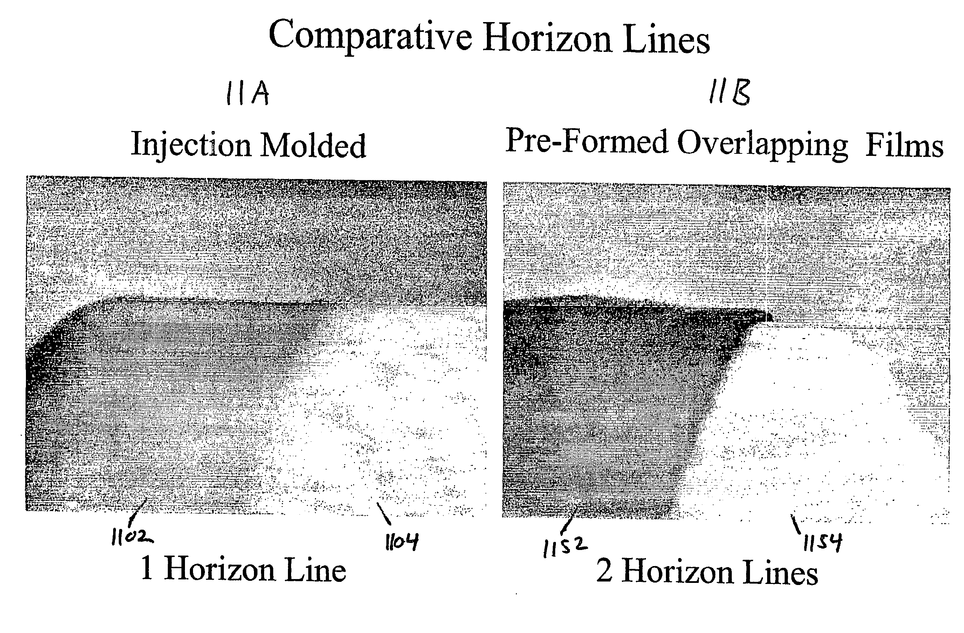

The advantageous nature of the present invention is further illustrated by Figs. 11A and 11B. Fig. 11A is a micrograph of first and second shell portions 1102 and 1104, respectively, of an injection molded dosage form of this invention. As shown in Fig. 11A, the interface of shell portions 1102 and 1104 is a flat seam in which the transition from first shell portion 1102 to second shell portion 1104 lies along a single "horizon line" (shown as a dashed line in Fig. 11A). In contrast, Fig. 11B depicts a prior art dosage form made from pre-formed overlapping films to form a first shell portion 1152 and second shell portion 1154, as shown. As is clear from Fig. 11B, the prior art dosage form exhibits a "step" at the interface of first shell portion 1152 and second shell portion 1154, thereby producing separate "horizon lines" (shown as dashed lines in Fig. 11B). Such a step is disadvantageous because it is less visually elegant than a smooth surface and because the step can be felt on the tongue and may be considered abrasive or unpleasant by the consumer. Additionally, the uneven surface or step may get caught in packaging machinery, reducing througbput/efficiency; or the uneven surface or step can serve as an opening point from which one shell layer can be removed either purposely or unintentionally, reducing the tamper-evidence of the dosage form.

In another preferred embodiment, the interface of the first and second shell portions is a flat seam in which the transition from first shell portion to second shell portion lies along a single "horizon line."

This invention will be further illustrated by the following examples.

### Example 1

A series of tablets having a molded gelatin coating thereon were made according to the invention as follows:

Part A: Compressed tablets
The following ingredients were mixed well in a plastic bag: 89.4 parts acetaminophen USP (590 mg/tablet) and 8.0 parts of synthetic wax X-2068 T20 (53 mg/tablet). Next, 2.1 parts of sodium starch glycolate (EXPLOTAB) (13.9 mg/tablet) and 0.09 parts of silicon dioxide (0.6 mg/tablet) were added to the bag, and mixed well. Then 0.36 parts of magnesium stearate NF (2.4 mg/tablet) were added to the bag, and the ingredients were again mixed. The resulting dry blend was compressed into tablets on a compression module as described in copending U.S. Application Serial No. 09/966,509 at pages 16-27. using 11mm (7/16) inch extra deep concave tablet tooling. The compression module was a double row, rotary apparatus, comprising a fill zone, insertion zone, compression zone, ejection zone, and purge zone as shown in Figure 6 of U.S. Application Serial No. 09/966,509. The dies of the compression module were filled using vacuum assistance, with mesh screen filters located in die wall ports of each die. The resulting tablets (cores) had an average weight of 660 mg, thickness of 8mm (0.306 inches), and hardness of 31.4N (3.2 kp).

Part B: Tablet Coating
The tablets from Part A were conveyed to a thermal cycle molding module as described in copending U.S. Application Serial No. 09/966,497 at pages 27-51 via a transfer device as described in copending U.S. Application Serial No. 09/966,414 at pages 51-57.
The tablets were coated with red gelatin on one half thereof, and yellow gelatin on the other half thereof to form a shell.

The thermal cycle molding module, which applied the shell to the tablets, was of the type shown in Figure 28A of copending U.S. Application Serial No. 09/966,939. The mold units 204 of the thermal cycle molding module comprised upper mold assemblies 214, rotatable center mold assemblies 212 and lower mold assemblies 210 as shown in Figure 28C. Tablets were transferred to the mold assemblies, which then closed over the tablets. Shell flowable material, which was heated to a flowable state in reservoir 206, was injected into the mold cavities created by the closed mold assemblies. The temperature of the shell flowable material was then decreased, hardening it. The mold assemblies opened and ejected the coated cores. Coating was performed in two steps, each half of the tablets being coated separately as shown in the flow diagram of Figure 28B of copending U.S. Application Serial No. 09/966,939 via rotation of the center mold assembly.

The red gelatin coating was made as follows: purified water (450 g), Opatint Red DD-1761 (4.4 g), and Opatint Yellow DD-2125 (1.8 g) were mixed at room temperature till uniform. 275 Bloom Pork Skin Gelatin (150 g) and 250 Bloom Bone Gelatin (150 g) were added together in a separate container. The dry gelatin granules were manually stirred to mix. The purified water / Opatint solution was added to the gelatin granules, and mixed for about 1 minute to completely wet the gelatin granules. The gelatin slurry was placed in a water bath and heated to 55°C to melt and dissolve the gelatin. The gelatin solution was held at 55°C for approximately 3 hours (holding times at this temperature can generally range between about 2 and about 16 hours). The solution was then mixed until uniform (about 5 to 15 minutes), and transferred to a jacketed feed tank equipped with a propeller-type electric mixer. The gelatin solution was maintained at 55°C with continuous mixing during its use in the thermal cycling molding module.

The yellow gelatin coating was made as follows: purified water (450 g), and Opatint Yellow DD-2125 (6.2 g) were mixed at room temperature till uniform. 275 Bloom Pork Skin Gelatin (150 g) and 250 Bloom Bone Gelatin (150 g) were added together in a separate container. The dry gelatin granules were stirred manually to mix. The purified water / Opatint solution was added to the gelatin granules, and mixed for about 1 minute to completely wet the gelatin granules. The gelatin slurry was placed in a water bath and heated to 55°C to melt and dissolve the gelatin. The gelatin solution was held at 55°C for approximately 3 hours (holding times at this temperature can generally range between about 2 and about 16 hours). The solution was then mixed until uniform (about 5 to 15 minutes), and transferred to a jacketed feed tank equipped with a propeller-type electric mixer. The gelatin solution was maintained at 55°C with continuous mixing during its use in the thermal cycling molding module.

### Example 2

Coating thickness was measured for samples of the following tablets:
A. Extra Strength TYLENOL GelTabs (available from McNeil - PPC)
B. EXCEDRIN Migraine Geltabs (available from Bristol-Myers Squibb)
C. Tablets produced according to Example 1
Referring to the locations on the dosage form shown in Figure 2 (Location 1: center of first major face, t_{c1}; Locations 2 and 3: edges (near punch land) of intersection between first major face and side, t_{c2} and t_{c3}; Location 4: center of second major face, t_{c4}; and Locations 5 and 6: edges (near punch land) of intersection between second major face and side, t_{c5} and t_{c6}), the results are shown in Table 1 below:

**TABLE 1**

| | A | B | C |
|---|---|---|---|
| average coating thickness at major faces (locations 1,4) for 6 tablets | 145.17 µm | 220.40 µm | 195.37 µm |
| variability in coating thickness at major faces (locations 1,4) for 6 tablets | 10.12% | 5.01% | 8.79% |
| average coating thickness (locations 1-6 for 6 tablets) | 85 µm | 244.83 µm | 209.62 µm |
| coating thickness variability (RSD for locations 1-6 for 6 tablets) | 52.71% | 12.64% | 18.49% |
| average coating thickness at edges | 54.92 µm | 257.05 µm | 216.74 µm |
| coating thickness variability at edges (RSD for locations 2,3,5,6 for 6 tablets) | 19.80 | 11.88 | 20.56 |
| average difference in coating thickness between major face and edge (location 1-location2, location 4-location5) | 63.25% | 16.99% | 15.93% |
| maximum difference in coating thickness between major face and edge (location 1-location2, location 4-location5) | 72% | 33.4% | 40.6% |
| minimum difference in coating thickness between major face and edge (location 1-location2, location 4-location5) | 54% | 7.1% | 4.1% |

Thicknesses and diameters of 20 coated tablets from each of the three samples were also measured. The results are summarized in Table 2 below:

**TABLE 2**

| | A | B | C |
|---|---|---|---|
| average coated tablet thickness at major faces (across locations 1,4) for 20 tablets | 7.67mm | 6.55 mm | 7.99 mm |
| variability in coated tablet thickness at major faces (locations 1,4) for 20 tablets | 0.407% | 1.44% | 0.292% |
| average coated tablet diameter (across locations 7,8 for 20 tablets) | 11.46 mm | 12.58 mm | 11.74 mm |
| variability in coated tablet diameter (rsd across locations 7,8 for 20 tablets) | 0.183% | 0.476% | 0.275% |

### Example 3

A flowable material suitable for coating a compressed dosage form was made as follows:

| Material | % w/w |
|---|---|
| PEG 1450 (part 1) | 30.0 |
| PEG 1450 (part 2) | 30 - 50% |
| Polyethylene Oxide 300,000 | 15.0-25% |
| Glycerin | 0 - 10% |
| Red color solution* (3%w/w) | 5 |

| | |
|---|---|
| *Red color solution composition was 4.85% w/w Propylene Glycol and 0.15% w/w Red #40 dye | |

Polyethylene glycol (PEG) 1450 (part 1) and polyethylene oxide (PEO) 300,000 were shaken in a plastic bag until powders were mixed evenly. The 4.7L (5 qt) bowl of a planetary mixer (Hobart Corp., Dayton, OH) was heated to 80°C by circulating hot water. PEG 1450 (part 2) was poured into the bowl and melted to form a liquid. The color solution, and optionally, the glycerin were added while mixing at low speed. The PEG/PEO powder mixture was added and the mixture mixed for 15 minutes. The resulting mixture was allowed to stand in the Hobart bowl for 2 hours while maintaining the temperature at 80°C. Cast films (approximately 0.8mm thick) were prepared using a stainless steel mold (51mm (2") x 127mm (5") x 0.8mm). The solution was transferred to a jacketed beaker (80°C) and de-aerated by vacuum for 6 hours. A second film was prepared using the same mold.

Increasing PEO from 15 to 25% (with corresponding decrease in PEG from 85 to 75%) increased yield stress (maximum force per unit area which can be applied before the film will deform permanently), and increased strain (% film elongation at break point).

Decreasing glycerin from 10% to 2% increased tensile strength (force per unit area required to break the film). Deaerating the glycerin-containing films prior to casting generally decreased tensile strength.

The flowable material may be applied using a thermal cycle molding module as described in copending U.S. Application Serial No. 09/966,497 at pages 27-51.

### Example 4

Another flowable material suitable for coating a compressed dosage form was made as follows:

| Material | %w/w |
|---|---|
| PEG 1450 granular | 70 - 75% |
| Polyethylene Oxide 600,000 | 15% |
| White beeswax | 5 - 10% |
| Red color solution* (3%w/w) | 5 |

| | |
|---|---|
| *Red color solution composition was 4.85% w/w Propylene Glycol and 0.15% w/w Red #40 dye | |

The 4.7L (5 qt) bowl of a planetary mixer (Hobart Corp., Dayton, OH) was heated to 80°C by circulating hot water. PEG 3350 granular was poured into the bowl and melted to form a liquid. The white beeswax, color solution, and polyethylene oxide were added while mixing at low speed. The resulting mixture was mixed for a total of 12 minutes, then allowed to stand in the Hobart bowl for 2 hours while maintaining the temperature at 80°C. Cast films were prepared using a glass slide. The solution was transferred to a jacketed beaker (80°C) and de-aerated by vacuum for 6 hours. A second film was prepared using the same mold.

The white beeswax formula had increased tensile strength compared to the glycerin formulas.

Examples 3 and 4 illustrate suitable formulations for the flowable material. Advantageously, these formulations are solvent (including water) free. This eliminates the need to evaporate solvent from coatings made from such formulations, shortening and simplifying drying. Accordingly, in one embodiment of the invention, the flowable material is substantially solvent-free, that is contains less than about 1 weight percent solvent, and preferably contains solvent.

The flowable material may be applied using a thermal cycle molding module as described in copending U.S. Application Serial No. 09/966,497 at pages 27-51.

### Example 5: Surface Gloss Measurement of Coated Tablets

Dosage forms made according to Example 1 were tested for surface gloss using an instrument available from TriCor Systems Inc. (Elgin, IL) under the tradename TRI-COR MODEL 805A/806H SURFACE ANALYSIS SYSTEM and generally in accordance with the procedure described in "TriCor Systems WGLOSS 3.4 Model 805A/806H Surface Analysis System Reference Manual" (1996), except as modified below.

This instrument utilized a CCD camera detector, employed a flat diffuse light source, compared tablet samples to a reference standard, and determined average gloss values at a 60 degree incident angle. During its operation, the instrument generated a gray-scale image, wherein the occurrence of brighter pixels indicated the presence of more gloss at that given location.

The instrument also incorporated software that utilized a grouping method to quantify gloss, i.e., pixels with similar brightness were grouped together for averaging purposes.

The "percent full scale" or "percent ideal" setting (also referred to as the "percent sample group" setting), was specified by the user to designate the portion of the brightest pixels above the threshold that will be considered as one group and averaged within that group. "Threshold," as used herein, is defined as the maximum gloss value that will not be included in the average gloss value calculation. Thus, the background, or the non-glossy areas of a sample were excluded from the average gloss value calculations. The method disclosed in K. Fegley and C. Vesey, "The Effect of Tablet Shape on the Perception of High Gloss Film Coating Systems," which is available at www.colorcon.com as of 18 March, 2002 was used to minimize the effects resulting from different tablet shapes, and to report a metric that was comparable across the industry. (The 50% sample group setting was selected as the setting which best approximated analogous data from tablet surface roughness measurements.)

After initially calibrating the instrument using a calibration reference plate (190-228; 294 degree standard; no mask, rotation 0, depth 0), a standard surface gloss measurement was then created using gel-coated caplets available from McNeil-PPC, Inc. under the tradename Extra Strength TYLENOL Gelcaps. The average gloss value for a sample of 112 of such gel-coated caplets was then determined, while employing the 25 mm full view mask (190-280), and configuring the instrument to the following settings:
Rotation: 0
Depth: 6mm (0.25 inches)
Gloss Threshold: 95
% Full Scale: 50%
Index of Refraction: 1.57

The average surface gloss value for the reference standard was determined to be 269.

Each sample of coated tablets was then independently tested in accordance with the same procedure.

A 50-tablet sample prepared according to the method of Example 1 possessed an average surface gloss of 241 gloss units on the yellow faces and 248 gloss units on the red faces.

Additional samples of other, commercially available coated tablets were also tested in accordance with the same procedure and compared to the same standard. The results are summarized in Table A below:

**Table A: Gloss values of commercially available coated tablets**

| Product | MOTRIN IB * Caplet (white) | EXCEDRIN ** Aspirin free Caplets (red) | EXCEDRIN ** Migraine Geltab (green side) | EXCEDRIN ** Migraine Geltab (white side) | Extra Strength TYLENOL Geltabs * (yellow side) | Extra Strength TYLENOL Geltabs * (red side) |
|---|---|---|---|---|---|---|
| Type of coating | sprayed film | sprayed film | gelatin enrobed | gelatin enrobed | dipped | Dipped |
| No. of tablets tested | 41 | 40 | 10 | 10 | 112 | 112 |
| Gloss Value(gl oss units) | 125 | 119 | 270 | 264 | 268 | 268 |

| | | | | | | |
|---|---|---|---|---|---|---|
| * Available from McNEIL-PPC, Inc. ** Available from Bristol-Myers Squibb, Inc. | | | | | | |

This example showed that the dosage forms of the present invention possessed a high surface gloss value (e.g. 241-248 gloss units in this example) that either was comparable or superior to that possessed by commercially available gelatin-coated tablets. In contrast, typical sprayed films possessed a substantially lower surface gloss, e.g. 119 to 125 gloss units in this example.

### Example 6: Moisture Uptake Comparison

Tablets prepared according to the method of Example 1 herein were compared with commercially available gelatin coated tablets for their moisture uptake properties. The comparitor samples were selected to represent the prior art methods of dipping (Extra Strength TYLENOL Gelcap from McNeil-PPC Inc.) and enrobing (all other samples, as detailed in Table B below), which have been discussed previously herein.

Samples were evaluated using a Dynamic Vapor Sorption (DVS) analysis instrument (DVS-2000, available from Surface Measurement Systems Ltd., London, UK). The DVS system is a controlled environmental compartment with a precise microbalance. The weight change of the sample at various relative humidities was recorded over time.

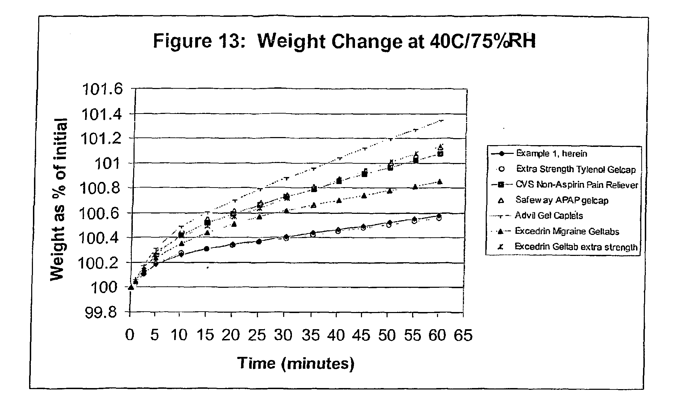

Each product was tested by placing three gelatin coated tablets in the DVS system at 40°C and 75% RH for 1 hour. The weight change was recorded. The balance was equilibrated at the test conditions and tared prior to sample testing. The sampling rate was set to collect the weight at intervals of 20 seconds. Figure 13 shows detailed results. The relative mass after 60 minutes at 40°C and 75% RH (expressed as a percent of the initial mass) is depicted in Table B below.

**Table B**

| Sample | Source | Relative mass (%) |
|---|---|---|
| Example 1 | - | 100.577 |
| Extra Strength TYLENOL Acetaminophen Gelcap | McNeil-PPC Inc | 100.557 |
| CVS Non-Aspirin Pain Reliever Geltabs | CVS | 101.074 |
| Safeway Acetaminophen Gelcap | Safeway | 101.125 |
| ADVIL Gel Caplets | Wyeth | 101.347 |
| EXCEDRIN Migraine Geltabs | Bristol-Myers Squibb | 100.851 |
| EXCEDRIN Geltab extra strength | Bristol-Myers Squibb | 101.134 |

The moisture uptake of the product from Example 1 at 60 minutes of exposure to 40°C/75% RH was about 0.58%, compared to a moisture uptake of about 0.85% for the enrobed product, and a moisture uptake of about 0.56% for the dipped product. These results indicate that the product of the present invention absorbs less moisture over time than products made by prior art enrobing methods, and are similar in moisture absorption properties to gelatin-dipped products. The dosage forms of the present invention will demonstrate superior physical stability to enrobed dosage forms when exposed to similar humidity conditions over longer periods of time.

## Claims

1. A dosage form comprising a core having an outer surface and a shell having outer and inner surfaces, wherein the shell surrounds the core, the dosage form moisture uptake at 60 minutes of exposure to 40°C and 75% relative humidity is less than 0.80%, the shell thickness is in the range of 100-400 µm, the relative standard deviation of the shell thickness on the dosage form is less than 30%, the dosage form contains less than 1 % by weight of charge agents selected from metal salicylates; quaternary ammonium salts; benzalkonium chloride; benzethonium chloride; trimethyl tetradecyl ammonium bromide (cetrimide); and cyclodextrins and their adducts; the shell is free of a raised seam, the inner surface of the shell has indentations and protrusions corresponding inversely to indentations and protrusions on the outer surface of the core wherein the indentations and protrusions have a length, width, height or depth greater than 10 µm; the finished shell of the dosage form contains at least 80% of a material selected from film formers, gelling polymers (hydrocolloids), low-melting hydrophobic materials, non-crystallizable sugars, and mixtures thereof, and the shell is formed by injection molding.

2. The dosage form of Claim 1, wherein the shell has a surface gloss value of at least 175 gloss units, wherein a gloss unit is a measure of reflected light determined according to the method set forth in Example 5.

3. The dosage form of Claim 2, wherein the shell has a surface gloss value of at least 210 gloss units.

4. The dosage form of Claim 1, wherein the shell comprises a first shell portion and a second shell portion which are joined at an interface.

5. The dosage form of Claim 4, wherein the core is a tablet having a major face and the total thickness of the shell at the interface is not greater than the thickness of the thickest at the center of the major tablet face.

6. The dosage form of Claim 4, wherein the first and second shell portions form an interlocking pattern at the interface.

7. The dosage form of Claim 1, wherein the shell has a pore volume of less than 0.02 cm³/g of pores having a pore diameter of 0.5-5.0 µm.

## Patentansprüche

1. Darreichungsform, die einen Kern mit einer Außenfläche und eine Hülle mit Außenund Innenflächen umfasst, wobei die Hülle den Kern umschließt, die Feuchtigkeitsaufnahme der Darreichungsform nach 60 Minuten bei 40°C und 75% relativer Feuchte weniger als 0,80% beträgt, die Hüllendicke im Bereich von 100-400 µm liegt, die relative Standardabweichung der Hüllendicke auf der Darreichungsform geringer als 30% ist, die Darreichungsform weniger als 1 Gew.-% Ladungsagentien enthält, ausgewählt aus Metallsalicylaten; quartären Ammoniumsalzen; Benzalkoniumchlorid; Benzethoniumchlorid; Trimethyltetradecylammoniumbromid (Cetrimid); und Cyclodextrinen und deren Addukten; die Hülle frei von einer erhabenen Naht ist, die Innenfläche der Hülle Einkerbungen und Vorsprünge aufweist, die umgekehrt Einkerbungen und Vorsprüngen auf der Außenfläche des Kerns entsprechen, wobei die Einkerbungen und Vorsprünge eine Länge, Breite, Höhe oder Tiefe von mehr als 10 µm besitzen; die fertige Hülle der Darreichungsform wenigstens 80% eines Materials enthält, das ausgewählt ist aus Filmbildnern, gelierenden Polymeren (Hydrokolloiden), niedrigschmelzende hydrophobe Materialien, nichtkristallisierbaren Zuckern und Mischungen davon, und die Hülle durch Spritzguss hergestellt ist.

2. Darreichungsform nach Anspruch 1, **dadurch gekennzeichnet, dass** die Hülle einen Oberflächenglanzwert von wenigstens 175 Glanzeinheiten besitzt, wobei eine Glanzeinheit ein Maß für reflektiertes Licht ist, bestimmt gemäß dem in Beispiel 5 angegebenen Verfahren.

3. Darreichungsform nach Anspruch 2, **dadurch gekennzeichnet, dass** die Hülle einen Oberflächenglanzwert von wenigstens 210 Glanzeinheiten besitzt.

4. Darreichungsform nach Anspruch 1, **dadurch gekennzeichnet, dass** die Hülle einen ersten Hüllenteil und einen zweiten Hüllenteil umfasst, die an einer Grenzfläche verbunden sind.

5. Darreichungsform nach Anspruch 4, **dadurch gekennzeichnet, dass** der Kern eine Tablette mit einer Hauptfläche ist und die Gesamtdicke der Hülle an der Grenzfläche nicht größer ist als die Dicke der dicksten Stelle in der Mitte der Haupttablettenfläche.

6. Darreichungsform nach Anspruch 4, **dadurch gekennzeichnet, dass** die ersten und zweiten Hüllenteile ein ineinandergreifendes Muster an der Grenzfläche bilden.

7. Darreichungsform nach Anspruch 1, **dadurch gekennzeichnet, dass** die Hülle ein Porenvolumen von weniger als 0,02 cm²/g Poren mit einem Porendurchmesser von 0,5-5,0 µm besitzt.

## Revendications

1. Forme pharmaceutique comprenant un noyau ayant une surface externe et une enveloppe ayant des surfaces externe et interne, dans laquelle l'enveloppe entoure le noyau, le recaptage d'humidité de la forme pharmaceutique à 60 minutes d'exposition à 40°C et 75 % d'humidité relative est inférieur à 0,80 %, l'épaisseur de l'enveloppe est dans la plage allant de 100 à 400 µm, la déviation standard relative de l'épaisseur de l'enveloppe sur la forme pharmaceutique est inférieure à 30 %, la forme pharmaceutique contient moins de 1 % en poids d'agents de charge choisis parmi les salicylates métalliques ; les sels d'ammonium quaternaire ; le chlorure de benzalconium ; le chlorure de benzéthonium ; le bromure de triméthyltétra-décylammonium (cétrimide) ; et les cyclo-dextrines et leurs adduits ; l'enveloppe est exempte d'une bordure en relief, la surface interne de l'enveloppe a des indentations et des protubérances correspondant inversement aux indentations et protubérances sur la surface externe du noyau dans lequel les indentations et les protubérances ont une longueur, une largeur, une hauteur ou une profondeur supérieure à 10 µm ; l'enveloppe finie de la forme pharmaceutique contient au moins 80 % d'un composé choisi parmi les filmogènes, les polymères gélifiants (hydrocolloïdes), les composés hydrophobes à faible point de fusion, les sucres non cristallisables, et des mélanges de ceux-ci, et l'enveloppe est formée par moulage par injection.

2. Forme pharmaceutique selon la revendication 1, dans laquelle l'enveloppe a une valeur de brillance superficielle d'au moins 175 unités de brillance, dans laquelle une unité de brillance est une mesure de la lumière réfléchie déterminée selon la méthode exposée dans l'exemple 5.

3. Forme pharmaceutique selon la revendication 2, dans laquelle l'enveloppe a une valeur de brillance superficielle d'au moins 210 unités.

4. Forme pharmaceutique selon la revendication 1, dans laquelle l'enveloppe comprend une première partie d'enveloppe et une deuxième partie d'enveloppe qui sont liées à une interface.

5. Forme pharmaceutique selon la revendication 4, dans laquelle le noyau est un comprimé ayant une face principale et l'épaisseur totale de l'enveloppe à l'interface n'est pas supérieure à l'épaisseur de la partie la plus épaisse au centre de la face principale du comprimé.

6. Forme pharmaceutique selon la revendication 4, dans laquelle les première et deuxième parties de l'enveloppe forment un motif d'imbrication à l'interface.

7. Forme pharmaceutique selon la revendication 1, dans laquelle l'enveloppe a un volume de pores inférieur à 0,02 cm³/g de pores ayant un diamètre de pores de 0,5 à 5,0 µm.
